# EUROPÄISCHE PATENTSCHRIFT

(11) **EP 1 631 372 B1**
(45) Veröffentlichungstag und Bekanntmachung des Hinweises auf die Patenterteilung: **05.05.2010**
(21) Anmeldenummer: 04739297.2
(22) Anmeldetag: 21.05.2004
(51) Int. Cl.: B01F 17/00, C11D 17/00, C11D 3/37

(54) **EIN TENSID UND EIN COTENSID UMFASSENDE MISCHUNG**
MIXTURE COMPRISING A SURFACTANT AND A COSURFACTANT
MELANGE COMPRENANT UN TENSIO-ACTIF ET UN CO-TENSIO-ACTIF

(30) Priorität: 22.05.2003 DE 10323178
(43) Veröffentlichungstag der Anmeldung: 08.03.2006
(73) Patentinhaber: BASF SE, 67056 Ludwigshafen (DE)
(72) Erfinder: MOCK-KNOBLAUCH, Cordula, 67063 Ludwigshafen (DE); WAGNER, Norbert, 67112 Mutterstadt (DE); OETTER, Günter, 67227 Frankenthal (DE); VÖLKEL, Ludwig, 67117 Limburgerhof (DE); STEINMETZ, Bernhard, 67117 Limburgerhof (DE); DYLLICK-BRENZINGER, Rainer, 67346 Speyer (DE); SCHRÖDER, Jörg, 69469 Weinheim (DE); PETROVIC, Susanne, 67063 Ludwigshafen (DE)
(86) Internationale Anmeldenummer: PCT/EP2004/005518
(87) Internationale Veröffentlichungsnummer: WO 2004/103542

(56) Entgegenhaltungen:
- US-A- 5 045 311
- US-A- 5 932 630
- US-B1- 6 506 485

## Beschreibung

Die Erfindung betrifft eine Mischung, umfassend ein Tensid und ein Cotensid, eine Verwendung einer Mischung zum Stabilisieren von Emulsionen, ein Verfahren zur Herstellung eines amphiphilen Polymers, eine Mikroemulsion, enthaltend ein Tensid und ein Cotensid, eine Verwendung einer Mischung oder einer Mikroemulsion sowie Wasch-, Reinigungs-, Desinfektions-, Netz-, Beschichtungs-, Klebe-, Lederentfettungs-, Feuchthalte- oder Textilbehandlungsmittel oder pharmazeutische, Pflanzenschutz- oder kosmetische Formulierung, insbesondere Sonnenschutz-, Hautpflege- oder Haarstylingmittel, Duschgele, Shampoos, Badezusätze oder Duftöle.

Tenside sind Substanzen, die die Grenzflächenspannung zwischen miteinander nicht mischbaren flüssigen Phasen, einer polaren Phase, häufig Wasser und einer unpolaren, organischen Phase herabsetzen und somit ihre gegenseitige Löslichkeit erhöhen. Tenside verfügen über einen charakteristischen Aufbau und weisen mindestens eine hydrophile und eine hydrophobe Struktureinheit auf. Dieser Aufbau wird auch als amphiphil bezeichnet.

Tenside sind ökologisch besonders relevante Stoffe, deren Umweltverträglichkeit sichergestellt werden muss. Neben einer guten Abbaubarkeit von Tensidrückständen in Abwässern ist es daher besonders wichtig, die eingesetzten Tensidmengen möglichst ohne Beeinträchtigung ihrer Wirksamkeit herabzusetzen, das heißt die Effizienz der Tenside zu steigern. Als Tensideffizienz wird dabei üblicherweise die Menge an Tensid bezeichnet, die benötigt wird, um einen bestimmten Effekt zu erzielen, zum Beispiel um den Anteil unpolarer Phase in der polaren Phase bzw. umgekehrt zu solubilisieren oder um die Obeflächenspannung bei möglichst niedriger Konzentration möglichst stark zu verringern.

Übliche konventionelle Emulsionen können Öl- und Wasserphase in sehr unterschiedlichen Volumenanteilen enthalten. Sie haben eine kontinuierliche und eine disperse Phase, die als sehr kleine, durch Belegung mit Tensiden stabilisierte Kügelchen in der kontinuierlichen Phase vorliegt. Je nach der Natur der kontinuierlichen Phase spricht man von Öl-in-Wasser-oder Wasser-in-Öl-Emulsionen. Diese Emulsionen sind im Idealfall kinetisch stabil, d. h. sie bleiben auch längere Zeit, aber nicht unbegrenzt, erhalten. Insbesondere bei Temperaturschwankungen können sie zur Phasentrennung durch Absitzen, Aufrahmen, Verdicken oder Flocken neigen.

Sogenannte Mikroemulsionen sind thermodynamisch stabile, fluide, optisch klare Formulierungen zweier nicht mischbarer Flüssigkeiten, wie Öl und Wasser. Mikroemulsionen entstehen, wenn ein Tensid, oder häufiger eine Mischung aus einem Tensid und einem Cotensid die Grenzflächenspannung Öl/Wasser auf extrem niedrige Werte häufig im Bereich -10⁻³ bis 10⁻⁹, bevorzugt 10⁻⁴ bis 10⁻⁶ N/m absenkt, so dass durch die thermische Bewegung homogen die beiden unlöslichen Phasen allein dispergiert bleiben. Mikroemulsionen weisen häufig bikontinuierliche Strukturen auf mit Gleichgewichtsbereichen, sogenannten Sub-Phasen in der Größenordnung von 100 bis 1000 Angström (vgl. Advanced Materials, 2000, 12, Nr. 23, Seiten 1751 ff.).

Bikontinuierliche Mikroemulsionen enthalten zwei Phasen, eine Wasser- und eine Ölphase, in Form von ausgedehnten nebeneinanderliegenden und ineinander verschlungenen Domänen, an deren Grenzfläche stabilisierende grenzflächenaktive Tenside in einer monomolekularen Schicht angereichert sind. Bikontinuierliche Mikroemulsionen bilden sich sehr leicht, in der Regel wegen der sehr niedrigen Grenzflächenspannung spontan, wenn die Einzelkomponenten, Wasser, Öl und ein geeignetes grenzflächenaktives System, vermischt werden. Da die Domänen in mindestens einer Dimension nur sehr geringe Ausdehnungen in der Größenordnung von Nanometern haben, erscheinen die Mikroemulsionen visuell transparent und sind je nach dem eingesetzten grenzflächenaktiven System in einem bestimmten Temperaturbereich thermodynamisch, das heißt zeitlich unbegrenzt, stabil.

Bikontinuierliche Mikroemulsionen sind zum Beispiel in dem Artikel "Mikroemulsionen - eine wissenschaftliche und anwendungstechnische Fundgrube?" von H.-F. Eicke in SÖFW-Journal 118 (1992), Seiten 311 bis 314, beschrieben.

Zum Erreichen der erforderlichen niedrigen Grenzflächenspannung an den Phasengrenzen enthalten die Mikroemulsionen spezielle Amphiphile, das heißt grenzflächenaktive Mittel, und in ihrer wässrigen Phase häufig gelöste Elektrolyte und gegebenenfalls weitere Hilfsstoffe. Elektrolyte werden vor allem dann zugesetzt, wenn die Amphiphile zum Teil oder ausschließlich ionische Tenside sind.

Aus DE-A 198 39 054 ist es bekannt, die Effizienz von Tensiden durch Zugabe von Additiven zu steigern, wobei als Additive AB-Blockcopolymere mit einem wasserlöslichen Block A und einem wasserunlöslichen Block B eingesetzt werden. Die Blöcke A und B können dabei Molekulargewichte zwischen 500 und 60.000 g/mol annehmen. Als Block A wird bevorzugt ein Polyethylenoxid-Block eingesetzt, allgemein jedoch alle wasserlöslichen Blöcke, die in Verbindung mit Block B ein Amphiphil bilden. Für den Block B werden Polymerisate eines einzigen Monomers oder eines Monomerengemisches beschrieben.

Die beschriebenen Blockcopolymere haben jedoch insbesondere den Nachteil, dass sie nach Herstellungsverfahren erhältlich sind, die für den Labormaßstab, nicht jedoch für den großtechnischen Einsatz geeignet sind. Die genannte Druckschrift verweist zum Herstellungsverfahren auf die DE-A 196 34 477, worin die Polymerisation unter Verwendung von Alkalimetallorganylen beschrieben wird, das heißt eine für den großtechnischen Einsatz ungeeignete Herstellungsmethode.

Aus US-B1 6,506,485 sind oberflächenaktive Substanzen bekannt, ausgewählt aus der Gruppe nicht-ionischer Alkylethylenoxide, Alkyl- und Arylpolyethylenoxide, Polyethylenoxid-Polypropylenoxid-Blockcopolymere und Sorbitanpolyethylenoxidmolekülen. Die Polypropylenoxide können den Pluronic^{®}Marken der BASF SE entsprechen, die Polyethylenoxid-Polypropylenoxid-Polyethylenoxid-Triblockcopolymere sind oder vom inversen Typ sein, d.h. reversed Polypropylenoxid-Polyethylenoxid-Polypropylenoxid-Triblockcopolymere (Pluronic-R^{®}). Tenside vom Pluronic^{®}-Typ sind jedoch stets Triblockcopolymere, d.h. sie umfassen stets drei Alkylenoxid-Blöcke.

Die US-A 5,045,311 beschreibt Mikroemulsionen für den Pflanzenschutz, enthaltend Phosphorsäureester und/oder -thioester als aktive Komponente und daneben eine nicht-ionische oberflächenaktive Substanz, vorzugsweise ein Blockcopolymer mit einem Molekulargewicht zwischen 1000 und 10000 sowie eine weitere oberflächenaktive Substanz ("cosurfactant"). Die nicht-ionische oberflächenaktive Substanz ist bevorzugt ein Blockcopolymer aus Polyethylenoxid und Polypropylenoxid, ein Alkylphenolpolyglykolether oder ein ethoxylierter Fettalkohol.

Es war demgegenüber Aufgabe der Erfindung, Substanzen zur Verfügung zu stellen, die als Cotenside zur Steigerung der Effizienz von Tensiden in Emulsionen, insbesondere in Mikroemulsionen, einsetzbar sind, und die in wirtschaftlich vorteilhafter Weise, auf der Basis von großtechnischen Ausgangssubstanzen sowie auf großtechnisch realisierbaren Umsetzungswegen erhalten werden können. Insbesondere soll eine Steigerung der Effizienz von Tensiden in bikontinuierlichen Mikroemulsionen erreicht werden.

Die Lösung besteht in einer Mischung, enthaltend ein Tensid und ein Cotensid, die dadurch gekennzeichnet ist, dass man als Cotensid ein amphiphiles Polymer mit der allgemeinen Strukturformel 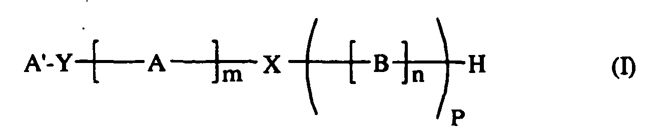 einsetzt, worin
- A': ein unverzweigter oder verzweigter Alkyl-, Cycloalkyl-, Aryl- oder Aralkylrest mit 8 bis 60 C-Atomen,
- Y: O oder S,
- A: eine Struktureinheit mit der Formel 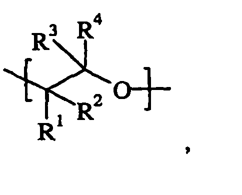
worin
- R¹, R², R³ und R⁴: unabhängig voneinander die Substituenten Wasserstoff, Methyl, Ethyl, n-Propyl, iso-Propyl, Oktyl- oder Phenyl sind, mit der Ein- schränkung, dass höchstens drei der Substituenten R¹, R², R³ und R⁴ Wasserstoff sind,
- m: eine Laufzahl im Bereich von 10 bis 300,
- X: eine Struktureinheit mit der Formel 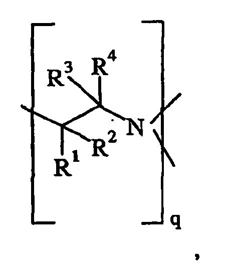
worin die Substituenten
- R¹, R², R³ und R⁴: unabhängig voneinander jeweils Wasserstoff, Methyl, Ethyl, n- Propyl, iso-Propyl, Oktyl oder Phenyl sind,
q = 0 oder q = 1,
- B: eine monomere Untereinheit basierend auf Ethylenoxid oder einer Mischung aus Ethylenoxid und Propylenoxid,
- n: eine Laufzahl im Bereich von 20 bis 500 und
p = q + 1 ist.
Es wurde überraschend gefunden, dass sich amphiphile Polymere mit der oben definierten Struktur besonders gut als Cotenside eignen, indem sie die Effizienz von Tensiden steigern und dabei aus großtechnischen und somit preiswert zugänglichen Stoffen auf großtechnischen Umsetzungswegen erhältlich sind. Die erfindungsgemäßen amphiphilen Polymere sind in der Regel technische Gemische aus Substanzen mit einer mehr oder weniger breiten Molekulargewichtsverteilung.

Die allgemeine Strukturformel umfasst somit sowohl lineare Strukturen, wenn q = 0 ist, als auch in y-Form verzweigte Strukturen, wenn q = 1 ist.

Die Struktureinheit A'-Y ist ein hydrophober Bauteil des Cotensids, und zwar ein monofunktioneller unverzweigter oder verzweigter Alkohol- oder Thiolrest, abgeleitet aus der Gruppe der C₁- bis C₆₀-Alkyl-, Cycloalkyl-, Aryl- oder Aralkylalkohole oder -thiole. Bevorzugt sind verzweigte oder unverzweigte Alkohole oder -thiole, mit 8 bis 30 C-Atomen pro Alkohol- oder Thiolrest.

Obwohl als Starter-Alkohole A'-OH grundsätzlich auch alle kürzerkettigen aliphatischen Monohydroxyalkohole mit 1 bis 5 Kohlenstoffatomen pro Molekül eingesetzt werden können, sind monofunktionelle aliphatische Alkohole mit 6 bis 18 Kohlenstoffatomen pro Molekül bevorzugt, besonders bevorzugt monofunktionelle aliphatische Alkohole mit 8 bis 15 Kohlenstoffatomen pro Molekühl.

Erfindungsgemäß geeignete Alkohole sind insbesondere Octanol, 2-Ethylhexanol, Nonanol, Decanol, Undecanol, Dodecanol, 2-Butyloctanol, Tridecanol, Tetradecanol, Pentadecanol, iso-Octanol, iso-Nonanol, iso-Decanol, iso-Undecanol, iso-Dodecanol, iso-Tridecanol, iso-Tetradecanol, iso-Pentadecanol bevorzugt iso-Decanol, 2-Propylheptanol, Tridecanol, i-soTredecanol oder Gemische aus C₁₃- bis C₁₅-Alkoholen oder Gemische von 2-Propylheptanol mit strukturisomeren C₁₀-Alkoholen. Oxoalkohole, wie sie üblicherweise durch Hydroformulierung von linearen oder verzweigten Olefinen mit 4 bis 29 Kohlenstoff, die zum Beispiel durch Oligomerisierung von Ethen, Propen, 1-Buten, isomeren Buten-Gemischen oder aus Gemischen der zuvor genannten Alkene hergestellt werden können, erhalten werden, oder von Alkoholen, wie sie ausgehend von Olefinen mit 5 bis 30 Kohlenstoffatomen entweder durch Ozonolyse und nachfolgende Reduktion oder durch Epoxidierung und nachfolgende Hydrolyse oder durch Halogenierung mit Chlor oder Brom und nachfolgende alkalische Hydrolyse erhalten werden, ableiten.

Beispielsweise kann es sich bei den erfindungsgemäß als Starterverbindung eingesetzten Alkoholen um Guerbet-Alkohole handeln, insbesondere Ethylhexanol, Propylheptanol, Butyloctanol. Daher betrifft die vorliegende Erfindung auch in einer besondere bevorzugten Ausführungsform ein Verfahren, wobei die Starterverbindung ein Guerbet-Allcohol ist.

Bei den als Starterverbindung eingesetzten Alkoholen kann es sich auch um Gemische verschiedener Isomere handeln.

Diese Gemische können sich aus den vorgenannten Alkoholen ableiten oder bei deren Herstellung anfallen, beispielsweise Rohprodukte und einzelne Fraktionen, wie sie bei der destillativen Aufarbeitung der Rohprodukte anfallen. Beispiele für geeignete Gemische sind sogenannte Oxoöle oder Oxoölfraktionen, wie sie bei der Herstellung von Oxoalkoholen regelmäßig anfallen.

Vorteilhaft kann als Starteralkohol A'-OH ein Alkoholgemisch eingesetzt werden, das durch Trimerisierung von Buten und anschließende Hydroformylierung und Hydrierung erhalten und als Tridecanol N bezeichnet wird.

Propylheptanol beispielsweise kann ausgehend von Valeraldehyd durch Aldolkondensation und nachfolgende Hydrierung erhalten werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 in Römpp, Chemie Lexikon, 9. Auflage, Stichwort "Aldol-Addition", Seite 91. Die Hydrierung des Aldolkondensationsproduktes folgt allgemeinen Hydrierbedingungen.

Des weiteren kann 2-Propylheptanol durch Kondensation von 1- Pentanol (als Mischung der entsprechenden Methylbutanole-1) in Gegenwart von KOH bei erhöhten Temperaturen hergestellt werden, siehe zum Beispiel Marcel Guerbet, C.R. Acad Sci Paris 128, 511, 1002 (1899). Des weiteren ist auf Römpp, Chemie Lexikon, 9. Auflage, Georg Thieme Verlag Stuttgart, und die dort genannten Zitate sowie Tetrahedron, Vol. 23, Seiten 1723 bis 1733, hinzuweisen.

Weiterhin sind auch Alkohole geeignet, die aus einer Addition von Aceton an Aldehyde mit nachfolgender Hydrierung entstehen, wie in DE-A 100 35 617 beschrieben. Geeignet sind auch Paraffin-Oxdidationsprodukte, die im wesentlichen sekundäre Alkohole darstellen (zum Beispiel von C_{12/14}-Paraffin-Gemischen oder C₁₀- bis C₁₆-Paraffin-Gemischen). Geeignete Alkohole sind auch zum Beispiel sekundäre Alkohole, die man durch saure Addition von Wasser an Olefine oder durch radikalische oder sonstige Oxidation von Olefinen erhält.

Nach den oben beschriebenen Verfahren sind auch eine Vielzahl von Handelsprodukten erhältlich, die häufig als Isomerenmischungen vorliegen und preiswert zur Verfügung stehen. Beispielsweise genannt seien das Produkt der Umsetzung von 2-Ethylhexanal mit Aceton oder Methylethylketon und abschließender Hydrierung, das Produkt der Umsetzung von C_{13/15}-Aldehyd mit Aceton oder Methylethylketon, das Produkt der Umsetzung eines Isomerengemisches unterschiedlicher C₁₃-Aldehyde, von sogenanntem iso-Tridecanal mit Aceton oder Methylethylketon. Beispiele für Starter-Alkohole, die durch Addition von Wasser am α-Olefinen erhältlich sind, sind 2-Decanol, 2-Dodecanol, 2-Tetradecanol oder 2-Hexadecanol.

Als Starter-Alkohole A'-OH eignen sich weiterhin alicyclische und aromatische Hydroxyverbindungen, vorzugsweise Verbindungen der Formeln 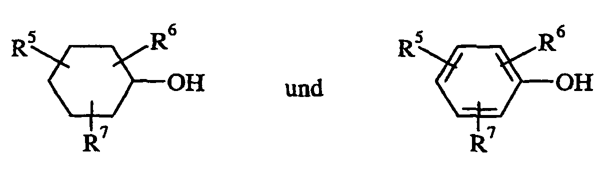 worin
R⁵, R⁶ und R⁷ unabhängig voneinander für Wasserstoff oder C₁-C₃₀-Alkyl stehen.

Bevorzugte alicyclische und aromatische Hydroxyverbindungen sind Cyclohexanol, Phenol, die Kresol-Isomere, Isobutylphenol, Isobutylkresol, Düsobutylphenol, Düsobutylkresol, tert.-Butylphenol, tert.-Butylkresol, Di-tert.-butylphenol, Di-tert.-butylkresol, Isooctylphenol, Düsooctylphenol, Isononylphenol, Düsononylphenol, Isododecylphenol, Düsododecylphenol und Mischungen davon.

Die hydrophobe Struktureinheit A ist bevorzugt aus einem oder mehreren der nachfolgenden Monomere: Propenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 2,3-Hexenoxid, 3,4-Hexenoxid, 2-Methyl-1,2-pentenoxid, 2-Ethyl-1,2-butenoxid, 3-Methyl-1,2-pentenoxid, 4-Methyl-1,2-pentenoxid, Decenoxid, 4-Methyl-1,2-pentenoxid, Styroloxid oder aus Gemischen hiervon gebildet. Dazu zählen vorzugsweise auch durch Oxidation großtechnisch zugänglicher Olefinströme erhältliche Gemische, die weitere von den zuvor genannten verschiedene Alkylenoxide und/oder von der Oxidation nicht erfasste Olefine und/oder Inerte (Alkane) enthalten können.

Die Laufzahl m, die die Anzahl der sich wiederholenden Struktureinheiten A bezeichnet, nimmt bevorzugt einen Wert im Bereich von 50 bis 250, insbesondere von 60 bis 160, an.

Die Struktureinheit X, enthaltend eine Aminogruppe, kann als Verzweigungsstelle in das amphiphile Polymer eingebaut werden.

Die Struktureinheit [B]ₙ ist ein hydrophiler Bauteil des Cotensids, gebildet aus sich sich wiederholenden Ethylenoxid- oder Ethylenoxid-/Propylenoxid-Einheiten. Dabei ist B eine monomere Untereinheit, basierend auf Ethylenoxid oder auf einer Mischung aus Ethylenoxid (EO) und Propylenoxid (PO). Die Struktureinheit [B]ₙ kann ein statistisches Copolymer, ein Gradientcopolymer, ein alternierendes oder ein Blockcopolymer aus EO und PO sein.

Die Polymerstruktur kann ein einziges hydrophiles Bauteil [B]ₙ oder aber, über die Verzweigungsstelle am Stickstoffatom, zwei hydrophile Bauteile [B]ₙ umfassen.

Die Laufzahl n, die die Anzahl der sich wiederholenden Struktureinheiten B bezeichnet, nimmt bevorzugt einen Wert im Bereich zwischen 50 und 300 an.

Vorteilhaft kann B eine Ethylenoxid-/Propylenoxid-Mischung mit 0 bis 50 % Propylenoxid, vorzugsweise mit 5 bis 20 % Propylenoxid sein.

Die erfindungsgemäße Mischung umfasst neben den vorstehend beschriebenen Co-Tensiden ein Tensid. Dabei kann es sich auch um eine Mischung von Tensiden handeln. Grundsätzlich kann jedes Tensid, aus jeder der bekannten Tensid-Gruppen, insbesondere ionische oder nichtionische Tenside, oder auch Mischungen von ionischen oder nicht-ionischen Tensiden eingesetzt werden.

Geeignet sind, je nach Einsatzgebiet der erfindungsgemäßen Mischungen, beispielsweise alle klassischen Reiniger-Tenside, oder lebensmittel-zugelassene Tenside, wie Tweens® oder Spans®. Von den Tensidklassen her sind geeignet nicht-ionische, anionische, kationische, amphotere Tenside; insbesondere auch Polymertenside, Peptidtenside, Silikontenside, aminosäurebasierte Tenside, Zuckertenside, fettbasierte Tenside, Geminitenside, Aminoxide, Amidoaminoxide, Alkylbetaine, Ethercarboxylate, Ampho-Acetate, Alkylsulfate oder Sulfosuccinate.

Der Anteil des Cotensids, bezogen auf das Tensid, liegt bevorzugt im Bereich von 0,01 bis 99,99%, insbesondere zwischen 1 und 50 %, besonders bevorzugt zwischen 5 und 25 %.

Geeignete anionische Tenside sind beispielsweise Fettalkoholsulfate von Fettalkoholen mit 8 bis 22, vorzugsweise 10 bis 18 Kohlenstoffatomen, zum Beispiel C₉- bis C₁₁-Alkoholsulfate, C₁₂- bis C₁₃-Allcoholsulfate, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, das man zunächst einen C₈- bis C₂₂-, vorzugsweise einen C₁₀- bis C₁₈-Alkohol, zum Beispiel einen Fettalkohol, alkoxyliert und das Alkoxylierungsprodukt anschließend sulfatiert. Für die Alkoxylierung verwendet man vorzugsweise Ethylenoxid, wobei man pro Mol Fettalkohol 2 bis 50, vorzugsweise 3 bis 20 mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid enthalten. Die alkoxylierten C₈- oder bis C₂₂-Alkohole können die Ethylenoxid-, Propylenoxid-und butylenoxideinheiten in Form von Blöcken oder in statistischer Verteilung enthalten.

Geeignet sind auch Alkansulfonate, wie C₈- bis C₂₄-, vorzugsweise C₁₀- bis C₁₈-Alkansulfonate sowie Seifen, wie Na oder K-Salze von C₈- bis C₂₄-Carbonsäuren.

Weitere geeignete anionische Tenside sind N-Acylsarkosinate mit aliphatischen gesättigten oder ungesättigten C₈- bis C₂₅-Acylresten, vorzugsweise C₁₀- bis C₂₀-Acylresten, zum Beispiel N-Oleoylsarkosinat.

Weiterhin können die erfindungsgemäßen Mischungen C₁₀- bis C₁₃-lineare und/oder -leicht verzweigte Alkylbenzolsulfonate (LAS) enthalten.

Die anionischen Tenside werden der Mischung, vorzugsweise in Form von Salzen zugegeben. Geeignete Kationen in diesen Salzen sind Alkalimetallsalze wie Natrium, Kalium und Lithium und Ammoniumsalze wie zum Beispiel Hydroxyethylammonium-, Di(hydroxyethyl)ammonium- und Tri(hydroxyethyl)ammoniumsalze.

Geeignete nichtionische Tenside sind insbesondere:
- alkoxylierte C₈- bis C₂₂-Alkohole wie Fettalkoholalkoxylate oder Oxoalkoholalkoxylate. Diese können mit Ethylenoxid, Propylenoxid und/oder Butylenoxid alkoxyliert sein. Als Tenside einsetzbar sind hierbei sämtliche alkoxylierten Alkohole, die mindestens zwei Moleküle eines der vorstehend genannten Alkylenoxide addiert enthalten. Hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Die nichtionischen Tenside enthalten pro Mol Alkohol im allgemeinen 2 bis 50, vorzugsweise 3 bis 20 Mol mindestens eines Alkylenoxids. Vorzugsweise enthalten diese als Alkylenoxid Ethylenoxid. Die Alkohole haben vorzugsweise 10 bis 18 Kohlenstoffatome. Je nach Art des bei der Herstellung verwendeten Alkoxylierungskatalysators weisen die Alkoxylate eine breite oder enge Alkylenoxid-Homologenverteilung auf;
- Alkylphenolalkoxylate wie Alkylphenolethoxylate mit C₆- bis C₁₄-Akylketten und 5 bis 30 Alkylenoxideinheiten;
- Alkylpolyglucoside mit 8 bis 22, vorzugsweise 10 bis 18 Kohlenstoffatomen in der Alkylkette und im allgemeinen 1 bis 20, vorzugsweise 1,1 bis 5 Glucosideinheiten Sorbitanalkanoate, auch alkoxyliert;
- N-Albylglucamide, Fettsäurealkoxylate, Fettsäureaminalkoxylate, Fettsäureamidalkoxylate, Fettsäurealkanolamidalkoxylate, alkoxyliert, Blockcopolymere aus Ethylenoxid, Propylenoxid und/oder Butylenoxid, Polyisobuten-Ethoxylate, Polyisobuten-Maleinsäureanhydrid-Derivate, Monoglyceride, auch alkoxyliert sowie Bisglyceride.

Besonders geeignete nichtionische Tenside sind Alkylalkoxylate oder Gemische von Alkylalkoxylaten, wie sie beispielsweise in DE-A 102 43 363, DE-A 102 43 361, DE-A 102 43 360, DE-A 102 43 365, DE-A 102 43 366, DE-A 102 43 362 oder in DE-A 43 25 237 beschrieben sind. Hierbei handelt es sich um Alkoxyliemngsprodukte, die durch Umsetzung von Alkanolen mit Alkylenoxiden in Gegenwart von Alkoxylierungskatalysatoren erhalten wurden oder um Gemische von Alkoxylierungsprodukten. Besonders geeignete Starteralkohole sind die sogenannten Guerbet-Alkohole, insbesondere Ehtylhexanol, Propylheptanol und Butyloktanol. Besonders bevorzugt ist Propylheptanol. Bevorzugte Alkylenoxide sind Propylenoxid und Ethylenoxid, wobei Alkylalkoxylate mit direkter Anbindung eines bevorzugt kurzen Poylypropylenoxidblocks an den Starteralkohol, wie sie beispielsweise in DE-A 102 43 365 beschrieben sind, insbesondere aufgrund ihres geringen Restalkoholgehalts und ihrer guten biologischen Abbaubarkeit bevorzugt sind.

Als Alkoxylierungskatalysatoren können Basen eingesetzt werden, beispielsweise Alkalihydroxide oder Alkalialkoholate, jedoch auch Lewis-Säuren, beispielsweise BF₃, SbCl₅, SnCl₄ x 2H₂0, BF₃ x H₃BO₄, oder BF₃-Dietherat. Besonders geeignete Alkoxylierungskatalysatoren sind Doppelhydroxid-Tone wie Hydrotalkit, die insbesondere mit Additiven modifiziert sein können, wie in DE-A 43 25 237 beschrieben.

Je nach Wahl des Alkoxylierungskatalysators resultieren jeweils spezifische Eigenschaften der Alkoxylate, insbesondere bezüglich der Verteilung des Alkoxylierungsgrades. So werden bei Verwendung der letztgenannten Doppelhydroxid-Tone Alkoxylierungsprodukte mit einer engen Molekulargewichtsverteilung bzw. Homologenverteilung erhalten, die für den Einsatz in den erfindungsgemäßen Mischungen mit Cotensiden besonders geeignet sind.

Die vorstehend beschriebenen vorteilhaften Eigenschaften, insbesondere bezüglich des Alkoxylierungsgrades, werden auch durch Einsatz von Doppelmetallcyanitd (DMC)-Verbindungen erreicht, wie sie beispielsweise in DE-A 102 43 361 als Alkoxylierungskatalysatoren beschrieben sind.

Gegenstand der Erfindung ist auch ein Verfahren zur Herstellung eines amphiphilen Polymers mit der allgemeinen Strukturformel (I) 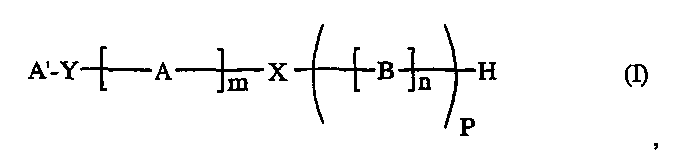 das dadurch gekennzeichnet ist, dass man einen einwertigen unverzweigten oder verzweigten Alkohol oder ein entsprechendes Thiol mit einem die Struktureinheit 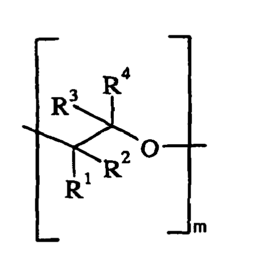 bildenden Monomer umsetzt, und
- die terminale OH-Gruppe unmittelbar mit Ethylenoxid oder einer Mischung aus Ethylenoxid und Propylenoxid oder
- die terminale OH-Gruppe zunächst zu einem primären oder sekundären Amin und anschließend mit Ethylenoxid oder einer Mischung aus Ethylenoxid und Propylenoxid umsetzt.

Das erfindungsgemäße Verfahren basiert auf großtechnisch zugänglichen Ausgangsstoffen, die durch großtechnisch einfach realisierbare Umsetzungen zu den gewünschten Cotensid-Strukturen führen.

Als Alkoxylierungskatalysatoren können Basen eingesetzt werden, beispielsweise Alkalihydroxide oder Alkalialkoholate, jedoch auch Lewis-Säuren, beispielsweise BF₃, SbCl₅, SnCl₄ x 2H₂0, BF₃ x H₃BO₄, oder BF₃-Dietherat. Besonders geeignete Alkoxylierungskatalysatoren sind Doppelhydroxid-Tone wie Hydrotalkit, die insbesondere mit Additiven modifiziert sein können, wie in DE-A 43 25 237 beschrieben.

Je nach Wahl des Alkoxylierungskatalysators resultieren jeweils spezifische Eigenschaften der Alkoxylate, insbesondere bezüglich der Verteilung des Alkoxylierungsgrades. So werden bei Verwendung der letztgenannten Doppelhydroxid-Tone Alkoxylierungsprodukte mit einer engen Molekulargewichtsverteilung bzw. Homologenverteilung erhalten, die für den Einsatz in den erfindungsgemäBen Mischungen als Cotensiden besonders geeignet sind.

Die vorstehend beschriebenen vorteilhaften Eigenschaften, insbesondere bezüglich des Alkoxylierungsgrades, werden auch durch Einsatz von Doppelmetallcyanid (DMC)-Verbindungen erreicht, wie sie beispielsweise in DE-A 102 43 361 als Alkoxylierungskatalysatoren beschrieben sind.

Gegenstand der Erfindung ist auch die Verwendung einer Mischung, umfassend ein Tensid und ein vorstehend beschriebenes Cotensid zum Stabilisieren von Emulsionen, insbesondere von Öl/Wasser-Emulsionen, Wasser/Öl-Emulsionen, Mikroemulsionen oder multiplen Emulsionen wie Öl/Wasser/Öl-Emulsionen oder Wasser/Öl/Wasser-Emulsionen. Stabilisieren bedeutet im vorliegenden Zusammenhang, dass durch Zusatz von Cotensiden die Effizienz von Tensiden gesteigert wird, das heißt die Solubilisierung eines definierten Öl/Wasser-Gemisches unter definierten Bedingungen mit einer geringeren Menge an Tensid ermöglicht wird.

Besonders bevorzugt eignen sich die vorstehend beschriebenen Cotenside zur Stabilisierung von Mikroemulsionen, das heißt zur Verschiebung des sogenannten X-Punktes, der die niedrigste Konzentration an Tensid bei gegebener Temperatur darstellt, ab der der thermodynamische Zustand der Mikroemulsion, das heißt der in makroskopischer Betrachtung einphasige Zustand, eintritt, zu niedrigeren Tensidkonzentrationen.

Die erfindungsgemäßen Mischungen sind grundsätzlich in allen Bereichen einsetzbar, in denen Emulsionen eine Rolle spielen, beispielsweise in den in DE-A 101 18 480 aufgeführten Anwendungsbereichen für Mischungen umfassend ein Tensid und ein AB-Blockcopolymerisat als Additiv (Cotensid), die daneben Zusatzstoffe enthalten, deren Effizienz durch das Tensid/Additivsystem gesteigert werden kann: Beispielsweise als Pflanzenstärkungs-, -wuchs- oder Pflanzenschutzmittel, Produkte mit mikrobioziden Wirkstoffen, Produkte mit positiv oder negativ wirkenden Mikroorganismen, insbesondere mit einem Gehalt an Enzymen, Reinigungs- und/oder Pflegemittel für den Haushalt und für gewerbliche Zwecke, Desinfektionsmittel, Haar-, Körperpflege- oder Reinigungsmittel, Fahrzeugreinigungs-, -pflege- und/oder -konservierungsmittel, Textilbehandlungsmittel, Leder- und/oder Pelzpflegemittel, als Farben, Lacke, Arzneimittel, Bauhilfsstoffe, Zahnpasten oder Mundspülmittel.
Synergistische Effekte, wie sie in DE-A 101 18 480 für das System Tensid/AB-Blockcopolymerisat in Verbindung mit zusätzlichen Bioziden, Mikroorganismen und/oder beliebigen anderen Wirkstoffen beschrieben sind, werden entsprechend für Systeme enthaltend die erfindungsgemäßen Mischungen umfassend ein Tensid und ein Cotensid sowie entsprechende Zusatzstoffe, insbesondere Biozide, Mikroorganismen und/oder beliebige andere Wirkstoffe, erreicht.

Gegenstand der Erfindung ist auch eine Mikroemulsion, enthaltend ein Tensid und ein Cotensid, die dadurch gekennzeichnet ist, dass man als Cotensid ein amphiphiles Polymer mit der allgemeinen Strukturformel 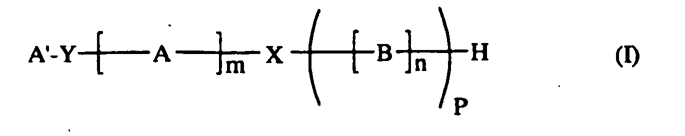 einsetzt, worin
- A': ein unverzweigter oder verzweigter Alkyl-, Cycloalkyl- Aryl- oder Aralkylrest mit 8 bis 60 C-Atomen,
- Y: O oder S,
- A: eine Struktureinheit mit der Formel 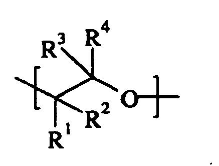
worin
- R¹, R², R³ und R⁴: unabhängig voneinander die Substituenten

Wasserstoff, Methyl, Ethyl, n-Propyl, iso-Propyl, Oktyl oder Phenyl sind, mit der Einschränkung, dass mindestens zwei und höchstens drei der Substituenten R¹, R², R³ und R⁴ Wasserstoff sind,
- X: m eine Laufzahl im Bereich von 10 bis 300, eine Struktureinheit mit der Formel 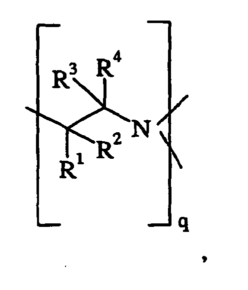 worin die Substituenten
- R¹, R², R³ und R⁴: unabhängig voneinander die Substituenten

Wasserstoff, Methyl, Ethyl, n-Propyl, iso-Propyl, Oktyl oder Phenyl sind,
q = 0 oder q = 1,
- B: ein monomere Untereinheit basierend auf Ethylenoxid oder einer Mischung aus E- thylenoxid und Propylenoxid,
- n: eine Laufzahl im Bereich von 20 bis 500 und
p = q + 1 ist.

Die Mikroemulsion ist grundsätzlich nicht eingeschränkt bezüglich des einsetzbaren Tensids oder Tensidgemisches. Bevorzugte Tenside sind vorstehend beschrieben.

Neben einem Tensid und einem wie oben definierten Cotensid enthält die Mikroemulsion ein polare Phase, in der Regel Wasser, sowie eine unpolare Phase, in der Regel einen oder mehrere Kohlenwasserstoffe.

Bevorzugt ist eine Mikroemulsion, umfassend ein Cotensid, worin A'-Y ein aliphatischer, alicyclischer, aromatischer oder aliphatisch-aromatischer, Monohydroxyalkohol- oder Thiolrest mit 8 bis 30 Kohlenstoffatomen pro Molekül ist.

Vorzugsweise umfasst die Mikroemulsion ein Cotensid, dessen Struktureinheit A aus einem oder mehreren der nachfolgenden Monomere: Propylenoxid, n-Butylenoxid, i-Butylenoxid, n-Pentenoxid, Decenoxid, Styroloxid oder aus einer Mischung aus Oxiden technisch verfügbarer Raffinatströme gebildet ist.

Vorzugsweise nimmt in den die Mikroemulsion bildenden Cotensiden die Laufzahl m einen Wert im Bereich von 50 bis 250, insbesondere von 60 bis 160, an.

Weiter bevorzugt nimmt in den die Mikroemulsion bildenden Cotensiden die Laufzahl n einen Wert im Bereich von 50 bis 300 an.

In den die Mikroemulsion bildenden Cotensiden ist B bevorzugt eine Ethylenoxid-/Propylenoxid-Mischung mit 0 bis 50 %, besonders bevorzugt mit 5 bis 20 % Propylenoxid.

Die erfindungsgemäßen Mischungen sind optimal geeignet für die Aufnahme und Abgabe von Hydrophoben, insbesondere die Verwendung als Waschmittel, Emulgator, Schaumregulierer, Netzmittel für harte Oberflächen oder als Reaktionsmedium für organische, anorganische, bioorganische oder photochemische Reaktionen.

Bevorzugt ist die Verwendung in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

Gegenstand der Erfindung sind auch Wasch-, Reinigungs-, Desinfektions-, Netz-, Beschichtungs-, Klebe-, Lederentfettungs-, Feuchthalte- oder Textilbehandlungsmittel oder pharmazeutische, Lebensmittel-, Pflanzenschutz- oder kosmetische Formulierung, insbesondere Sonnenschutz-, Hautpflege- oder Haarstylingmittel, Duschgele, Shampoos, Badezusätze oder Duftöle, enthaltend neben üblichen Inhaltsstoffen eine Mischung umfassend ein Tensid und ein wie vorstehend beschriebenes Cotensid oder eine Mikroemulsion umfassend ein Tensid und ein Cotensid.

Besonders geeignet ist der Einsatz der erfindungsgemäßen Mischungen in Zubereitungen für die Verwendung in der Kosmetik, Pharmazie sowie im Nahrungsmittelbereich, enthaltend mindestens ein Retinoid, mindestens ein wasserlösliches Antioxidans und mindestens ein öllösliches Oxidans, wie sie in der nicht vorveröffentlichten deutschen Patentanmeldung DE 102 337 40.3 beschrieben sind, deren Offenbarungsgehalt hiermit voll umfänglich in die vorliegende Patentanmeldung einbezogen wird. Hierbei handelt es sich um kosmetische sowie dermatologische oder pharmazeutische Zubereitungen, die in der Regel auf der Basis eines Trägers aufgebaut sind, der mindestens eine Ölphase enthält. Demgemäß kommen Öle, Cremes, Pasten oder fettfreie Gele oder bevorzugt Emulsionen in Betracht.

Die genannten Zubereitungen enthalten pro Gewichtsteil Retinoid mindestens ein Gewichtsteil eines oder mehrerer wasserlöslicher Antioxidantien und 0,1 bis 100 Gewichtsteile eines oder mehrerer öllöslicher Antioxidantien, wobei der Gehalt an einem oder mehreren wasserlöslichen Antioxidantien im Bereich von 0,05 bis 0,8 Gew.-% liegt, bezogen auf die Gesamtmenge der Zubereitungen.

Unter Retinoiden sind Vitamin A Alkohol (Retinol) und seine Derivate wie Vitamin A Aldehyd (Retinal), Vitamin A Säure (Retinsäure) und Vitamin A Ester wie Retinylacetat und Retinylpalmitat gemeint. Der Begriff Retinsäure umfasst dabei sowohl all-trans Retinsäure als auch 13-cis Retinsäure. Die Begriffe Retinol und Retinal umfassen beovzrugt die all-trans Verbindungen. Als bevorzugtes Retinoid verwendet man für die erfindungsgemäßen Zubereitungen all-trans-Retinol.

Als wasserlösliche Antioxidantien sind u.a. Ascorbinsäure, Natriumsulfit, Natriummetabisulfit, Natriumbisulfit, Natriumthiosulfit, Natriumformaldehydsulfoxylat, Isoascorbinsäure, Thioglycerin, Thiosorbit, Thioharnstoff, Thioglykolsäure, Cysteinhydrochlorid, 1,4-Diazobicyclo-(2,2,2)-oktan oder Mischungen davon gemeint.

Bevorzugte wasserlösliche Antioxidantien sind Ascorbinsäure (L-Ascorbinsäure) und Isoascorbinsäure (D-Ascorbinsäure), besonders bevorzugt L-Ascorbinsäure.

Bei der besonders bevorzugt verwendeten L-Ascorbinsäure kann es sich um die freie Säure aber auch um deren Salze handeln. Beispiele für Salze der L-Ascorbinsäure sind Alkali-oder Erdalkalimetallsalze der L-Ascorbinsäure wie Natrium-L-ascorbat, Kalium-L-ascorbat oder Calcium-L-ascorbat, aber auch Salze der L-Ascorbinsäure mit einer organischen A-minverbindung wie Cholinascorbat oder L-Carnitinascorbat. Ganz besonders bevorzugt verwendet man die freie L-Ascorbinsäure oder Natrium-L-ascorbat. Entsprechendes gilt für die Verwendung von D-Ascorbinsäure.

Als öllösliche Antioxidantien sind u.a. butyliertes Hydroxytoluol (BHT), Ascorbylpalmitat, butyliertes Hydroxyanisol, α-Tocopherol, Phenyl-α-naphthylamin oder Mischungen davon gemeint.

Bevorzugtes öllösliches Antioxidans ist α-Tocopherol, wobei es sich dabei sowohl um (R,R,R)- als auch um (all-rac)-α-Tocopherol handeln kann.

Zu den kosmetischen oder pharmazeutischen Zubereitungen kommen übliche Hilfsstoffe in Betracht, beispielsweise Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (zum Beispiel Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind unter anderem Bienenwachs, Paraffinwachs oder Mikrowachs gegebenenfalls in Kombination mit hydrophilen Wachsen zu nennen. Als Stabilisatoren können Metallsalze von Fettsäuren wie zum Beispiel Magnesium-, Aluminium- und/oder Zinkstearat eingesetzt werden. Geeignete Verdickungsmittel sind beispielsweise vernetzte Polyacrylsäuren und deren Derivate, Polysaccha ride, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pffanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservietungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkomission der Deutschen Forschungsgemeinschaft, veröffentlicht im Verlag Chemie, Weinheim 1984, zusammengestellt sind. Diese Farbstoffe werden üblicherweise in Konzentrationen von 0,001 bis 0,1 Gew.-%, bezogen auf die gesamte Mischung, eingesetzt.

Die erindungsgemäBen Mischungen können auch vorteilhaft in kosmetischen oder dermatologischen Zubereitungen eingesetzt werden, enthaltend mindestens einen UV-Filter, besonders bevorzugt in kosmetischen oder dermatologischen Zubereitungen enthaltend als UV-B-Filter 2,4,6-Trianilino-p-(carbo-2'-ethyl-hexyl-1'-oxi)-1,3,5-triazin, das von der BASF AG unter der Warenbezeichnung Uvinul® T150 vermarktet wird und als UV-A-Filter N,N-Diethylamino-hydroxybenzoyl-n-hexylbenzoat, das von der BASF AG unter der Warenbezeichnung Uvinul® A Plus vermarktet wird.

Besonders vorteilhaft können die erfindungsgemäßen Mischungen in kosmetischen oder dermatologischen Zubereitungen enthalten sein, wie sie in der nicht vorveröffentlichten deutschen Patentanmeldung DE 102 00 400 7885.8 beschrieben sind, die hiermit voll umfänglich in den Offenbarungsgehalt der vorliegenden Patentanmeldung einbezogen wird.

In der genannten Patentanmeldung wird eine Kombination aus Uvinul® T150, Uvinul® A Plus zusammen mit Zinkoxid und/oder Titandioxid beschrieben, wobei zusätzlich UV-A-und UV-B-Filtersubstanzen aus der nachfolgenden Tabelle enthalten sein können:

| **Nr.** | **Stoff** | **CAS-Nr.** **(= Säure)** |
|---|---|---|
| 1 | 4-Aminobenzoesäure | 150-13-0 |
| 2 | 3-(4'Trimethylammonium-benzylidenboman-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis (7,7-dimethyl-2-oxobicyclo[2.2.1]heptan-1-methansulfon- | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'-Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino] carbonyl]phenyl]amino]-1,3,5-triazin-2,4-diyl]diimino]bis-,bis(2-ethylhexyl)ester | 154702-15-5 |
| 30 | 2-(2H-Benzotriazol-2-yl)-4-methyl-6-[2-methyl-3-[1,3,3,3-tetramethyl-1-1[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexal)oxyl)] (Aniso Triazin) | 187393-00-6 |

Die Lichtschutzmittel enthaltenden kosmetischen und dermatologischen Zubereitungen sind in der Regel auf der Basis eines Trägers, der mindestens eine Ölphase enthält. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wässrige Lotionen.

Übliche Ölkomponenten in der Kosmetik sind beispielsweise Paraffinöl, Glycerylstearat, Isopropylmyristat, Düsopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure sowie Siliconöle.

Geeignete Siliconöle sind zum Beilspiel lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugt cyclische Siloxane weisen 4- bis 8-gliedrige Ringe auf. Geeignete cyclische Siloxane sind zum Beispiel unter der Bezeichung Cyclomethicon kommerziell erhältlich.

### Herstellungsbeispiel

Es wurde ein amphiphiles Polymer durch Umsetzung von C₁₃-Oxoakohol mit 100 Mol Butylenoxid/Mol C13-Oxoalkohol, in zwei Stufen und anschließende Umsetzung mit 164 Mol Ethylenoxid/Mol C13-Oxoalkohol wie nachstehend beschrieben hergestellt:

### Stufe 1 Umsetzung von C₁₃-Oxoalkohol mit 22 Mol Butylenoxid/Mol C₁₃-Oxoalkohol

50 g C₁₃-Oxoalkohol und 0,9 g Kalium-tert.-butylat wurden in einem 21 Metallreaktor vorgelegt und anschließend dreimal mit jeweils 5 bar Stickstoff inertisiert. Der Reaktorinhalt wurde auf 140°C erhitzt und anschließend 396 g Butylenoxid zudosiert. Es wurde bis zur Druckkonstanz nachgerührt. Nach dem Abkühlen und Entspannen des Reaktors und Entgasen am Rotationsverdampfer bei 80°C und 3 bis mbar wurden 445,5 g eines C₁₃-Oxoalkohols erhalten, der mit 22 Äquivalenten Butylenoxid/Mol alkoxyliert war.

### Stufe II Umsetzung von C₁₃-Oxoalkohol mit 78 Mol Butylenoxid/Mol C₁₃-Oxoalkohol

446 g C₁₃-Oxoalkoholbutoxylat aus Stufe I und 3,7 g Kalium-tert.-butylat wurden in einem 3,5 1 Metallreaktor vorgelegt und anschließend dreimal mit jeweils 5 bar Stickstoff inertisiert. Der Reaktorinhalt wurde auf 140°C erhitzt und anschließend 1404 g Butylenoxid zudosiert. Es wurde bis zur Druckkonstanz nachgerührt. Nach dem Abkühlen und Entspannen des Reaktores und Entgasen am Rotationsverdampfer bei 80°C und 3 bis 4 mbar wurden 1847,3 g eines C₁₃-Oxoalkohols erhalten, der mit 100 Äquivalenten Butylenoxid/Mol alkoxyliert war.

### Stufe III Umsetzung von C₁₃-Oxoalkoholbutoxylat mit 164 Mol Ethylenoxid/Mol C₁₃-Oxoalkohol

196 g des C₁₃-Oxoalkohol-Polybutoxylates aus Stufe II und 0,9 g Kalium-tert.-butylat wurden in einem 2 l Metallreaktor vorgelegt und anschließend dreimal mit jeweils 5 bar Stickstoff inertisiert. Der Reaktorinhalt wurde auf 120°C erhitzt und anschließend 190 g Ethylenoxid zudosiert. Es wurde bis zur Druckkonstanz nachgerührt. Nach dem Abkühlen und Entspannen des Reaktors und Entgasen am Rotationsverdampfer bei 80°C und 3 bis 4 mbar wurde ein Produkt mit folgender Zusammensetzung erhalten: 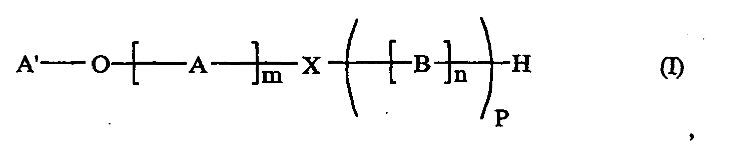 worin
- A'-O: ein C₁₃-Oxoalkohol,
- A: eine Struktureinheit der Formel 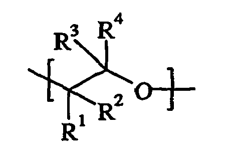 worin einer der Reste R¹ bis R⁴ ein Ethylrest und die übrigen drei Reste Wasserstoff sind,
- X: eine Struktureinheit der Formel 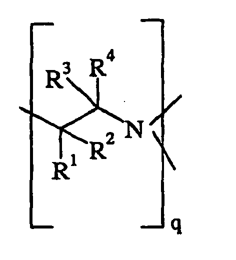 worin q = 0 ist und
- B: eine monomere Untereinheit basierend auf Ethylenoxid ist.

| | |
|---|---|
| OH-Zahl: | 13 mg KOH/g (Theorie: 10 mg KOH/g) |
| Basenzahl: | 0,4 mg KOH/g |

### Anwendungsbeispiel

In der nachfolgenden Figur ist die Verschiebung des X-Punktes, das heißt der Mindestkonzentration an Tensid bei gegebener Temperatur dargestellt, ab der für das Referenzsystem Wasser/n-Dekan und ein gegebenes Tensid (Lutensol® ON50 der BASF AG) die Wasser und die n-Dekan-Phase vollständig mischbar sind und eine thermodynamisch stabile Mikroemulsion entsteht.

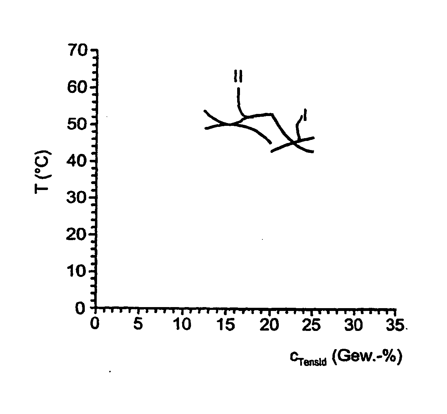

In der anliegenden Figur ist auf der Abszisse die Konzentration des Tensids Lutensol® ON50, in der Figur mit c_{Tensid,} in Gew.-% und auf der Ordinate die Temperatur in °C dargestellt. Ausschnitte aus den jeweiligen Phasendiagrammen ("Fisch"-Phasen-Diagramme) sind für das genannte Referenzsystem Wasser/n-Dekan 1 : 1 und das genannte Tensid Lutensol® ON50 unter I zum Vergleich, das heißt ohne Zusatz eines Cotensids und unter II für das erfindungsgemäße Anwendungsbeispiel mit Zusatz des vorstehend unter Herstellungsbeispiel beschriebenen Cotensids, in einer Konzentration von 10 %, bezogen auf das Tensid, dargestellt. Die Darstellung zeigt, dass sich der X-Punkt von 22,5 % Tensid im Vergleichsbeispiel zu 15 % Tensid im erfindungsgemäßen Beispiel mit Cotensid-Zusatz verschob.

### Anwendungsbeispiele für kosmetische oder dermatologische Zubereitungen

Zur Herstellung von kosmetischen oder dermatologischen Zubereitungen eingesetzt wurde ein Cotensid, das analog Stufe I aus dem vorstehend beschriebenen Herstellungsbeispiel durch Umsetzung von C13-Oxoalkohol mit 22 Mol Butylenoxid/Mol C13-Oxoalkohol und anschließende Umsetzung analog Stufe III des vorstehenden Herstellungsbeispiels, jedoch abweichend hiervon mit insgesamt 48 Mol Alkylenoxid, davon 0,95 % Ethylenoxid und 0,05 % Propylenoxid, erhalten wurde. Dieses amphiphile Polymer wird in der nachfolgenden Tabelle als Cotensid * bezeichnet.

Es wurden Emulsionen für kosmetische oder dermatologische Anwendungen nach folgender allgemeiner Vorschrift hergestellt:
Die jeweiligen Phasen A (ölhaltig) und B (wässrig) wurden getrennt auf ca. 85°C erwärmt. Phase A wurde langsam gerührt und Phase B in Phase A eingerührt, wobei die Mischtemperatur auf über 80°C gehalten wurde. Unter langsamem Rühren wurde auf Raumtemperatur abgekühlt.

Die Zusammensetzung der Phasen A und B für Beispiel 1 ist in der nachfolgenden Tabelle aufgeführt:

### Beispiel 1

| **Phase** | **Gew.-%** | **INCI** |
|---|---|---|
| **A** | 5,0 | Glyceryl Stearate, Ceteareth-20, Ceteareth-12, Cetearyl Alcohol, Cetyl Palmitate |
| | 1,0 | Ceteareth-20 |
| | 5,0 | Caprylic/Capric Triglyceride |
| | 5,0 | Mineral Oil |
| | 1,0 | Octyldodecanol |
| | 1,0 | Ethylhexyl Triazone (Uvinul® T150) |
| | 0,6 | Cotensid* |
| **B** | 5,0 | Glyzerin |
| | ad 100 | Aqua demin. |

Es wurde eine Mikroemulsion erhalten, die bei Raumtemperatur stabil war, sprühbar war und eine dynamische Viskosität von unter 50 mPa.s aufwies.

### Vergleichsbeispiel 1

Die Menge an Cotensid* wurde auf 0,4 Gew.-% abgesenkt. Es wurde keine stabile Mikroemulsion erhalten.

### Vergleichsbeispiel 2

Die Menge an Tensid (die ersten beiden Komponenten aus Phase A in der zu Beispiel 1 aufgeführten Tabelle) wurde von 6 Gew.-% auf 4 Gew.-% abgesenkt. Es wurde keine stabile Mikroemulsion erhalten.

## Patentansprüche

1. Mischung, enthaltend ein Tensid und ein Cotensid, **dadurch gekennzeichnet, dass** man als Cotensid ein amphiphiles Polymer mit der allgemeinen Strukturformel 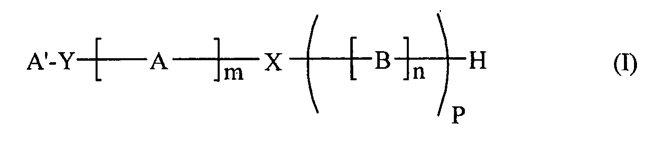 einsetzt, worin
A' ein unverzweigter oder verzweigter Alkyl- Cycloalkyl-, Aryl- oder Aralkylrest mit 8 bis 60 C-Atomen,
Y S oder O,
A eine Struktureinheit mit der Formel 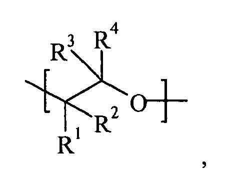 worin
R¹, R², R³ und R⁴ unabhängig voneinander die Substituenten Wasserstoff, Methyl, Ethyl, n-Propyl, iso-Propyl, Oktyl- oder Phenyl sind, mit der Einschränkung, dass höchstens drei der Substituenten R¹, R², R³ und R⁴ Wasserstoff sind,
m eine Laufzahl im Bereich von 10 bis 300,
X eine Struktureinheit mit der Formel 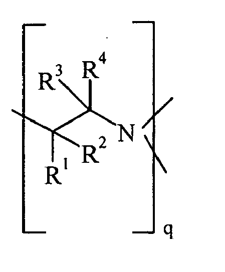 ist, worin die Substituenten
R¹, R², R³ und R⁴ unabhängig voneinander jeweils Wasserstoff, Methyl, Ethyl, n- Propyl, iso-Propyl, Oktyl oder Phenyl sind,
q = 0 oder q = 1,
B eine monomere Untereinheit basierend auf Ethylenoxid oder eine Mischung aus Ethylenoxid und Propylenoxid,
n eine Laufzahl im Bereich von 20 bis 500 und
p=q+ 1 ist.

2. Mischung nach Anspruch 1, **dadurch gekennzeichnet, dass** A'-Y ein monofunktioneller unverzweigter oder verzweigter Alkohol- oder Thiolrest mit 8 bis 30 Kohlenstoffatomen pro Molekül ist.

3. Mischung nach einem der Ansprüche 1 oder 2, **dadurch gekennzeichnet, dass** die Struktureinheit A aus einem oder mehreren der nachfolgenden Monomere: Propenoxid, 1-Butenoxid, 2,3-Butenoxid, 2-Methyl-1,2-propenoxid (Isobutenoxid), 1-Petnenoxid, 2,3-Pentenoxid 2-Methyl-1,2-butenoxid, 3-Methyl-1,2-butenoxid, 2,3-Hexenoxid 3,4-Hexenoxid, 2-Methyl-1,2-pentenoxid, 2-Ethyl-1,2-butenoxid, 3-Methyl-1,2-Pentenoxid, Decenoxid, 4-Methyl-1,2-pentenoxid, Styroloxid oder aus einer Mischung aus Oxiden technisch verfügbarer Raffinatströme gebildet ist.

4. Mischung nach einem der Ansprüche 1 bis 3, **dadurch gekennzeichnet, dass** die Laufzahl m einen Wert im Bereich von 50 bis 250, bevorzugt von 60 bis 160, annimmt.

5. Mischung nach einem der Ansprüche 1 bis 4, **dadurch gekennzeichnet, dass** die Laufzahl n einen Wert im Bereich zwischen 50 und 300 annimmt.

6. Mischung nach einem der Ansprüche 1 bis 5, **dadurch gekennzeichnet, dass** B eine Ethylenoxid-/Propylenoxid-Mischung mit 0 bis 50 %, bevorzugt 5 bis 20 % Propylenoxid ist.

7. Verfahren zur Herstellung eines amphiphilen Polymers mit der allgemeinen Strukturformel (I) 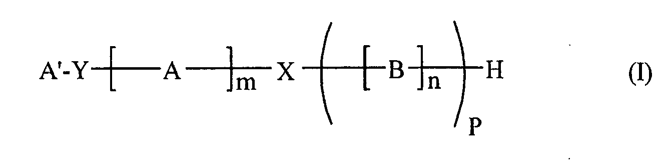 worin
A' ein unverzweigter oder verzweigter Alkyl- Cycloalkyl-, Aryl- oder Aralkylrest mit 8 bis 60 C-Atomen,
Y S oder O,
A eine Struktureinheit mit der Formel 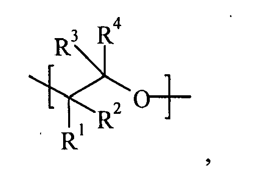 worin
R¹, R², R³ und R⁴ unabhängig voneinander die Substituenten
Wasserstoff, Methyl, Ethyl, n-Propyl, iso-Propyl, Oktyl- oder Phenyl sind, mit der Einschränkung, dass höchstens drei der Substituenten R¹, R², R³ und R⁴ Wasserstoff sind,
m eine Laufzahl im Bereich von 10 bis 300,
X eine Struktureinheit mit der Formel 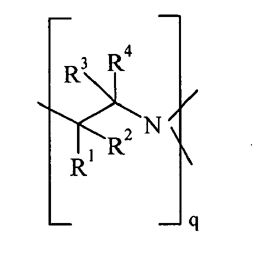 ist, worin die Substituenten
R¹, R², R³ und R⁴ unabhängig voneinander jeweils
Wasserstoff, Methyl, Ethyl, n-Propyl, iso-Propyl, Oktyl oder Phenyl sind,
q = 0 oder q = 1,
B eine monomere Untereinheit basierend auf Ethylenoxid oder eine Mischung aus Ethylenoxid und Propylenoxid,
n eine Laufzahl im Bereich von 20 bis 500 und
p=q+ 1 ist,
**dadurch gekennzeichnet, dass** man einen unverzweigten oder verzweigten Monohydroxyalkyl-, -aryl- oder -aralkylalkohol A'-OH oder ein entsprechendes Thiol A'-SH mit einem die Struktureinheit 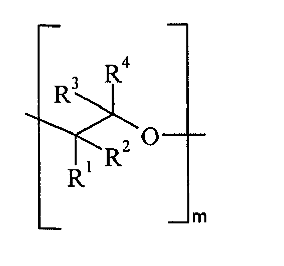 bildenden Monomer umsetzt, und
- die terminale OH-Gruppe unmittelbar mit Ethylenoxid oder einer Mischung aus Ethylenoxid und Propylenoxid oder
- die terminale OH-Gruppe zunächst zu einem primären oder sekundären Amin und anschließend mit Ethylenoxid oder einer Mischung aus Ethylenoxid und Propylenoxid umsetzt.

8. Verwendung einer Mischung nach einem der Ansprüche 1 bis 6 zum Stabilisieren von Emulsionen, bevorzugt von Mikroemulsionen.

9. Mikroemulsion, enthaltend ein Tensid und Cotensid, **dadurch gekennzeichnet, dass** man als Cotensid ein amphiphiles Polymer mit der allgemeinen Strukturformel 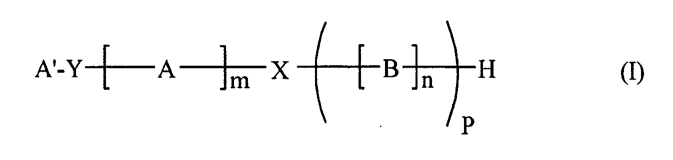 einsetzt, worin
A' ein unverzweigter oder verzweigter Alkyl-, Cycloalkyl-, Aryl oder Aralkylrest mit 8 bis 60 C-Atomen,
Y S oder O,
A eine Struktureinheit mit der Formel 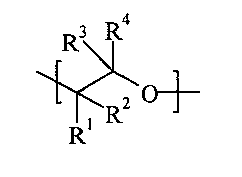 worin
R¹, R², R³ und R⁴ unabhängig voneinander die Substituenten Wasserstoff, Methyl, Ethyl, Propyl, Oktyl oder Phenyl sind, mit der Einschränkung, das mindestens zwei und höchstens drei der Substituenten R¹, R², R³ und R⁴ Wasserstoff sind,
m eine Laufzahl im Bereich von 10 bis 300,
X eine Struktureinheit mit der Formel 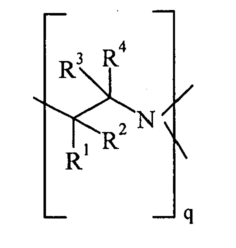 worin die Substituenten
R¹, R², R³ und R⁴ unabhängig voneinander die Substituenten
Wasserstoff, Methyl, Ethyl, Propyl, Oktyl oder Phenyl sind,
q = 0 oder q = 1,
B eine monomere Untereinheit basierend auf Ethylenoxid oder einer Mischung aus Ethylenoxid und Propylenoxid,
n eine Laufzahl im Bereich von 20 bis 500 und
p = q + 1 ist.

10. Mikroemulsion nach Anspruch 9, **dadurch gekennzeichnet, dass** A'-Y ein monofunktioneller unverzweigter oder verzweigter aliphatischer Alkohol oder Thiolrest mit 8 bis 30 Kohlenstoffatomen pro Molekül ist.

11. Mikroemulsion nach Anspruch 9 oder 10, **dadurch gekennzeichnet, dass** die Struktureinheit A aus einem oder mehreren der nachfolgenden Monomere: Propenoxid, 1-Butenoxid, 2,3-Butenoxid, 2-Methyl-1,2-propenoxid (Isobutenoxid), 1-Petnenoxid, 2,3-Pentenoxid 2-Methyl-1,2-butenoxid, 3-Methyl-1,2-butenoxid, 2,3-Hexenoxid 3,4-Hexenoxid, 2-Methyl-1,2-pentenoxid, 2-Ethyl-1,2-butenoxid, 3-Methyl-1,2-Pentenoxid, 4-Methyl-1,2-pentenoxid, Decenoxid, Styroloxid oder aus einer Mischung aus Oxiden technisch verfügbarer Raffinatströme gebildet ist.

12. Mikroemulsion nach einem der Ansprüche 9 bis 11, **dadurch gekennzeichnet, dass** die Laufzahl m einen Wert im Bereich von 50 bis 250, bevorzugt von 60 bis 160, annimmt.

13. Mikroemulsion nach einem der Ansprüche 9 bis 12, **dadurch gekennzeichnet, dass** die Laufzahl n einen Wert im Bereich zwischen 50 und 300 annimmt.

14. Mikroemulsion nach einem der Ansprüche 9 bis 13, **dadurch gekennzeichnet, dass** B eine Ethylenoxid-/Propylenoxid-Mischung mit 0 bis 50 %, bevorzugt mit 5 bis 20 % Propylenoxid ist.

15. Verwendung einer Mischung nach einem der Ansprüche 1 bis 6 oder einer Mikroemulsion nach einem der Ansprüche 9 bis 14 als Waschmittel, Emulgator, Schaumregulierer, Netzmittel für harte Oberflächen oder als Reaktionsmedium für organische, anorganische, bioorganische oder photochemische Reaktionen.

16. Verwendung nach Anspruch 15 in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

17. Wasch-, Reinigungs-, Netz-, Beschichtungs-, Klebe-, Lederentfettungs-, Feuchtehalte-oder Textilbehandlungsmittel oder pharmazeutische, Pflanzenschutz- oder kosmetische Formulierung, insbesondere Sonnenschutz-, Hautpflege- oder Haarstylingmittel, Duschgele, Shampoos, Badezusätze oder Duftöle, enthaltend neben üblichen Inhaltsstoffen eine Mischung nach einem der Ansprüche 1 bis 6 oder eine Mikroemulsion nach einem der Ansprüche 9 bis 14.

## Claims

1. A mixture comprising a surfactant and a cosurfactant, wherein the cosurfactant used is an amphiphilic polymer with the structural formula 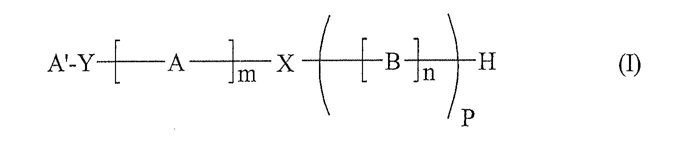 in which
A' is an unbranched or branched alkyl, cycloalkyl, aryl or aralkyl radical having 8 to 60 carbon atoms,
Y is S or O,
A is a structural unit with the formula 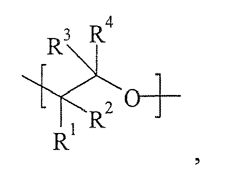 in which
R¹, R², R³ and R⁴ independently of one another, are the substituents hydrogen, methyl, ethyl, n-propyl, isopropyl, octyl or phenyl, with the restriction that at most three of the substituents R¹, R², R³ and R⁴ are hydrogen,
m is a running number in the range from 10 to 300,
X is a structural unit with the formula 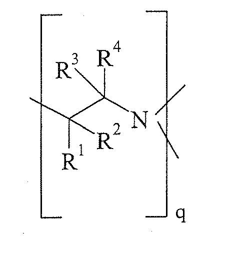 in which the substituents
R¹, R², R³ and R⁴ independently of one another, are each hydrogen, methyl, ethyl, n-propyl, isopropyl, octyl or phenyl,
q = 0 or q = 1,
B is a monomeric subunit based on ethylene oxide or a mixture of ethylene oxide and propylene oxide,
n is a running number in the range from 20 to 500 and
p = q + 1.

2. The mixture according to claim 1, wherein A'-Y is a monofunctional unbranched or branched alcohol or thiol radical having 8 to 30 carbon atoms per molecule.

3. The mixture according to either claim 1 or 2, wherein the structural unit A is formed from one or more of the following monomers: propene oxide, 1-butene oxide, 2,3-butene oxide, 2-methyl-1,2-propene oxide (isobutene oxide), 1-pentene oxide, 2,3-pentene oxide, 2-methyl-1,2-butene oxide, 3-methyl-1,2-butene oxide, 2,3-hexene oxide, 3,4-hexene oxide, 2-methyl-1,2-pentene oxide, 2-ethyl-1,2-butene oxide, 3-methyl-1,2-pentene oxide, decene oxide, 4-methyl-1,2-pentene oxide, styrene oxide or from a mixture of oxides of industrially available raffinate streams.

4. The mixture according to any of claims 1 to 3, wherein the running number m assumes a value in the range from 50 to 250, preferably from 60 to 160.

5. The mixture according to any of claims 1 to 4, wherein the running number n assumes a value in the range between 50 and 300.

6. The mixture according to any of claims 1 to 5, wherein B is an ethylene oxide/propylene oxide mixture containing 0 to 50%, preferably 5 to 20%, of propylene oxide.

7. A process for the preparation of an amphiphilic polymer with the structural formula (I) 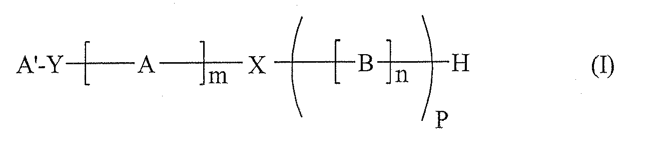 in which
A' is an unbranched or branched alkyl, cycloalkyl, aryl or aralkyl radical having 8 to 60 carbon atoms,
Y is S or O,
A is a structural unit with the formula 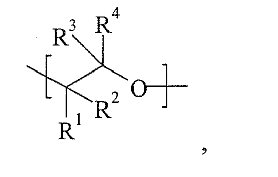 in which
R¹, R², R³ and R⁴ independently of one another, are the substituents hydrogen, methyl, ethyl, n-propyl, isopropyl, octyl or phenyl, with the restriction that at most three of the substituents R¹, R², R³ and R⁴ are hydrogen,
m is a running number in the range from 10 to 300,
X is a structural unit with the formula 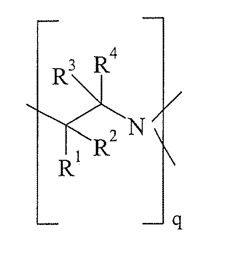 in which the substituents
R¹, R², R³ and R⁴ independently of one another, are each
hydrogen, methyl, ethyl, n-propyl, isopropyl, octyl or phenyl,
q = 0 or q = 1,
B is a monomeric subunit based on ethylene oxide or a mixture of ethylene oxide and propylene oxide,
n is a running number in the range from 20 to 500 and
p = q + 1,
which comprises reacting an unbranched or branched monohydroxyalkyl, -aryl or -aralkyl alcohol A'-OH or a corresponding thiol A'-SH with a monomer which forms the structural unit 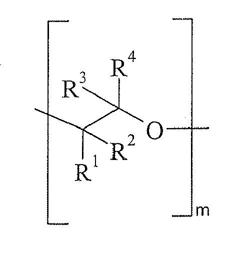 and reacting
- the terminal OH group directly with ethylene oxide or a mixture of ethylene oxide and propylene oxide or
- the terminal OH group firstly to give a primary or secondary amine and then with ethylene oxide or a mixture of ethylene oxide and propylene oxide.

8. The use of a mixture according to any of claims 1 to 6 for stabilizing emulsions, preferably microemulsions.

9. A microemulsion comprising a surfactant and cosurfactant, wherein the cosurfactant used is an amphiphilic polymer with the structural formula 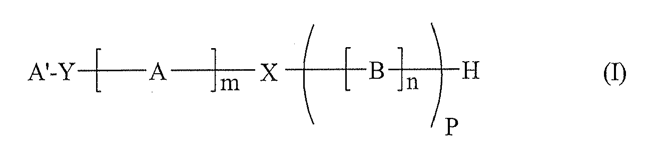 in which
A' is an unbranched or branched alkyl, cycloalkyl, aryl or aralkyl radical having 8 to 60 carbon atoms,
Y is S or O,
A is a structural unit with the formula 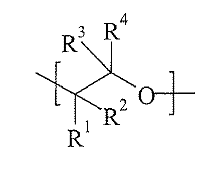 in which
R¹, R², R³ and R⁴ independently of one another, are the substituents hydrogen, methyl, ethyl, propyl, octyl or phenyl, with the restriction that at least two and at most three of the substituents R¹, R², R³ and R⁴ are hydrogen,
m is a running number in the range from 10 to 300,
X is a structural unit with the formula 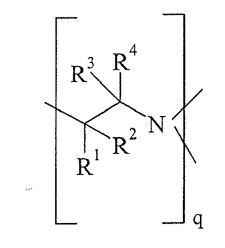 in which the substituents
R¹, R², R³ and R⁴ independently of one another, are the substituents
hydrogen, methyl, ethyl, propyl, octyl or phenyl,
q = 0 or q = 1,
B is a monomeric subunit based on ethylene oxide or a mixture of ethylene oxide and propylene oxide,
n is a running number in the range from 20 to 500 and
p = q + 1.

10. The microemulsion according to claim 9, wherein A'-Y is a monofunctional unbranched or branched aliphatic alcohol or thiol radical having 8 to 30 carbon atoms per molecule.

11. The microemulsion according to claim 9 or 10, wherein the structural unit A is formed from one or more of the following monomers: propene oxide, 1-butene oxide, 2,3-butene oxide, 2-methyl-1,2-propene oxide (isobutene oxide), 1-pentene oxide, 2,3-pentene oxide, 2-methyl-1,2-butene oxide, 3-methyl-1,2-butene oxide, 2,3-hexene oxide, 3,4-hexene oxide, 2-methyl-1,2-pentene oxide, 2-ethyl-1,2-butene oxide, 3-methyl-1,2-pentene oxide, 4-methyl-1,2-pentene oxide, decene oxide, styrene oxide or from a mixture of oxides of industrially available raffinate streams.

12. The microemulsion according to any of claims 9 to 11, wherein the running number m assumes a value in the range from 50 to 250, preferably from 60 to 160.

13. The microemulsion according to any of claims 9 to 12, wherein the running number n assumes a value in the range between 50 and 300.

14. The microemulsion according to any of claims 9 to 13, wherein B is an ethylene oxide/propylene oxide mixture containing 0 to 50%, preferably containing 5 to 20%, of propylene oxide.

15. The use of a mixture according to any of claims 1 to 6 or of a microemulsion according to any of claims 9 to 14 as detergent, emulsifier, foam regulator, wetting agent for hard surfaces or as reaction medium for organic, inorganic, bioorganic or photochemical reactions.

16. The use according to claim 15 in detergents, surfactant formulations for the cleaning of hard surfaces, humectants, cosmetic, pharmaceutical and crop protection formulations, paints, coatings, adhesives, leather degreasing compositions, formulations for the textile industry, fiber processing, metal processing, food industry, water treatment, paper industry, fermentation, mineral processing, fire protection or in emulsion polymerizations.

17. A detergent, cleaner, wetting agent, coating, adhesive, leather degreasing composition, humectant or textile treatment composition or a pharmaceutical, crop protection or cosmetic formulation, in particular sunscreen, skincare or hair styling composition, shower gel, shampoo, bath additive or scent oil, comprising, as well as customary ingredients, a mixture according to any of claims 1 to 6 or a microemulsion according to any of claims 9 to 14.

## Revendications

1. Mélange contenant un tensioactif et un co-tensioactif, **caractérisé en ce qu'**on utilise comme tensioactif un polymère amphiphile de formule développée générale 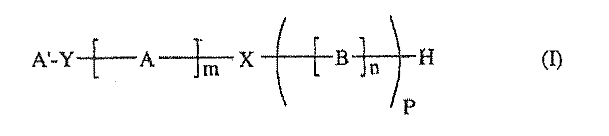 dans laquelle
A' représente un radical alkyle, cycloalkyle, aryle ou aralkyle, ramifié ou non ramifié, ayant de 8 à 20 atomes de carbone,
Y est un atome de soufre ou d'oxygène,
A représente un motif structural de formule 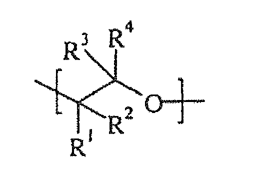 dans laquelle
R¹, R², R³ et R⁴ représentent, indépendamment les uns des autres, les substituants consistant en un atome d'hydrogène, un groupe méthyle, éthyle, n-propyle, isopropyle, octyle ou phényle, avec la restriction qu'au maximum trois des substituants R¹, R², R³ et R⁴ représentent des atomes d'hydrogène,
m est un nombre variable dans la plage de 10 à 300,
X représente un motif structural de formule 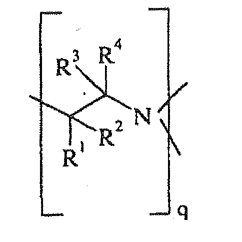 dans laquelle les substituants
R¹, R², R³ et R⁴ représentent chacun, indépendamment les uns des autres, un atome d'hydrogène, un groupe méthyle, éthyle, n- propyle, isopropyle, octyle ou phényle,
q = 0 ou q = 1,
B représente une sous-unité monomère à base d'oxyde d'éthylène ou d'un mélange d'oxyde d'éthylène et d'oxyde de propylène,
n est un nombre variable dans la plage de 20 à 500 et
p = q + 1.

2. Mélange selon la revendication 1, **caractérisé en ce que** A'-Y est un reste d'alcool ou de thiol monofonctionnel, ramifié ou non ramifié, ayant de 8 à 30 atomes de carbone par molécule.

3. Mélange selon la revendication 1 ou 2, **caractérisé en ce que** le motif structural A est constitué d'un ou plusieurs des monomères suivants : oxyde de propène, oxyde de 1-butène, oxyde de 2,3-butène, oxyde de 2-méthyl-1,2-propène (oxyde d'isobutène), oxyde de 1-pentène, oxyde de 2,3-pentène, oxyde de 2-méthyl-1,2-butène, oxyde de 3-méthyl-1,2-butène, oxyde de 2,3-hexène, oxyde de 3,4-hexène, oxyde de 2-méthyl-1,2-pentène, oxyde de 2-éthyl-1,2-butène, oxyde de 3-méthyl-1,2-pentène, oxyde de décène, oxyde de 4-méthyl-1,2-pentène, oxyde de styrène ou d'un mélange d'oxydes de courants de raffinats disponibles dans l'industrie.

4. Mélange selon l'une quelconque des revendications 1 à 3, **caractérisé en ce que** le nombre variable m a une valeur dans la plage de 50 à 250, de préférence de 60 à 160.

5. Mélange selon l'une quelconque des revendications 1 à 4, **caractérisé en ce que** le nombre variable n a une valeur dans la plage comprise entre 50 et 300.

6. Mélange selon l'une quelconque des revendications 1 à 5, **caractérisé en ce que** B est un mélange oxyde d'éthylène/oxyde de propylène comportant de 0 à 50 %, de préférence de 5 à 20 % d'oxyde de propylène.

7. Procédé pour la préparation d'un polymère amphiphile de formule développée générale (I) 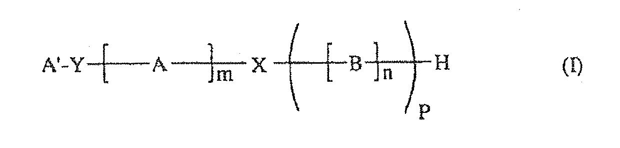 dans laquelle
A' représente un radical alkyle, cycloalkyle, aryle ou aralkyle, ramifié ou non ramifié, ayant de 8 à 60 atomes de carbone,
Y est un atome de soufre ou d'oxygène,
A représente un motif structural de formule 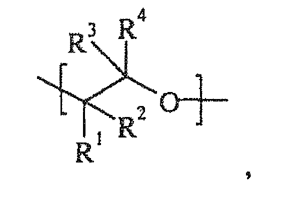
dans laquelle
R¹, R², R³ et R⁴ représentent, indépendamment les uns des autres, les substituants consistant en un atome d'hydrogène, un groupe méthyle, éthyle, n-propyle, isopropyle, octyle ou phényle, avec la restriction qu'au maximum trois des substituants R¹, R², R³ et R⁴ représentent des atomes d'hydrogène,
m est un nombre variable dans la plage de 10 à 300,
X représente un motif structural de formule 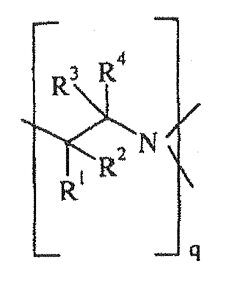 dans laquelle les substituants
R¹, R², R³ et R⁴ représentent chacun, indépendamment les uns des autres, un atome d'hydrogène, un groupe méthyle, éthyle, n- propyle, isopropyle, octyle ou phényle,
q = 0 ou q = 1,
B représente une sous-unité monomère à base d'oxyde d'éthylène ou d'un mélange d'oxyde d'éthylène et d'oxyde de propylène,
n est un nombre variable dans la plage de 20 à 500 et
p = q + 1,
**caractérisé en ce qu'**on fait réagir un alcool monohydroxyalkylique, -arylique ou -aralkylique, ramifié ou non ramifié, A'-OH ou un thiol A'-SH correspondant, avec un monomère formant le motif structural 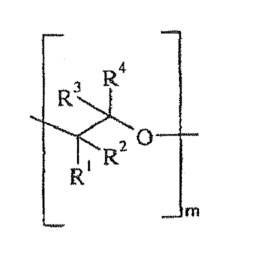 et
- on fait réagir le groupe OH terminal directement avec de l'oxyde d'éthylène ou un mélange d'oxyde d'éthylène et d'oxyde de propylène ou
- on convertit d'abord le groupe OH terminal en un groupe amino primaire ou secondaire et ensuite on le fait réagir avec de l'oxyde d'éthylène ou un mélange d'oxyde d'éthylène et d'oxyde de propylène.

8. Utilisation d'un mélange selon l'une quelconque des revendications 1 à 6, pour la stabilisation d'émulsions, de préférence de microémulsions.

9. Microémulsion, contenant un tensioactif et un co-tensioactif, **caractérisée en ce qu'**on utilise comme co-tensioactif un polymère amphiphile ayant la formule développée générale 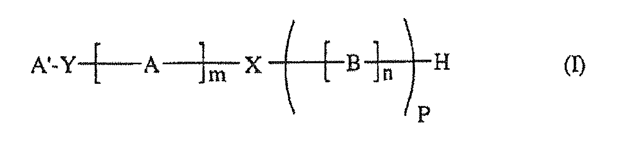 dans laquelle
A' représente un radical alkyle, cycloalkyle, aryle ou aralkyle, ramifié ou non ramifié, ayant de 8 à 60 atomes de carbone,
Y est un atome de soufre ou d'oxygène,
A représente un motif structural de formule 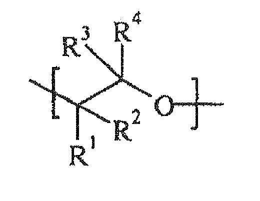 dans laquelle
R¹, R², R³ et R⁴ représentent, indépendamment les uns des autres, les substituants consistant en un atome d'hydrogëne, un groupe méthyle, éthyle, propyle, octyle ou phényle, avec la restriction qu'au moins deux et au maximum trois des substituants R¹, R², R³ et R⁴ représentent des atomes d'hydrogène,
m est un nombre variable dans la plage de 10 à 300,
X représente un motif structural de formule 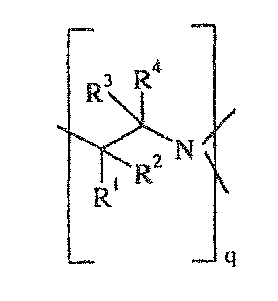 dans laquelle les substituants
R¹, R², R³ et R⁴ représentent, indépendamment les uns des autres, les substituants consistant en un atome d'hydrogène, un groupe méthyle, éthyle, propyle, octyle ou phényle,
q = 0 ou q = 1,
B représente une sous-unité monomère à base d'oxyde d'éthylène ou d'un mélange d'oxyde d'éthylène et d'oxyde de propylène,
n est un nombre variable dans la plage de 20 à 500 et
p = q + 1.

10. Microémulsion selon la revendication 9, **caractérisée en ce que** A'-Y est un reste d'alcool ou de thiol aliphatique monofonctionnel, ramifié ou non ramifié, ayant de 8 à 30 atomes de carbone par molécule.

11. Microémulsion selon la revendication 9 ou 10, **caractérisée en ce que** le motif structural A est constitué d'un ou plusieurs des monomères suivants : oxyde de propène, oxyde de 1-butène, oxyde de 2,3-butène, oxyde de 2-méthyl-1,2-propène (oxyde d'isobutène), oxyde de 1-pentène, oxyde de 2,3-pentène, oxyde de 2-méthyl-1,2-butène, oxyde de 3-méthyl-1,2-butène, oxyde de 2,3-hexène, oxyde de 3,4-hexène, oxyde de 2-méthyl-1,2-pentène, oxyde de 2-éthyl-1,2-butène, oxyde de 3-méthyl-1,2-pentène, oxyde de 4-méthyl-1,2-penténe, oxyde de décène, oxyde de styrène ou d'un mélange d'oxydes de courants de raffinats disponibles dans l'industrie.

12. Microémulsion selon l'une quelconque des revendications 9 à 11, **caractérisée en ce que** le nombre variable m a une valeur dans la plage de 50 à 250, de préférence de 60 à 160.

13. Microémulsion selon l'une quelconque des revendications 9 à 12, **caractérisée en ce que** le nombre variable n a une valeur dans la plage comprise entre 50 et 300.

14. Microémulsion selon l'une quelconque des revendications 9 à 13, **caractérisée en ce que** B est un mélange oxyde d'éthylène/oxyde de propylène comportant de 0 à 50 %, de préférence de 5 à 20 % d'oxyde de propylène.

15. Utilisation d'un mélange selon l'une quelconque des revendications 1 à 6 ou d'une microémulsion selon l'une quelconque des revendications 9 à 14, en tant que produit de lavage, émulsifiant, régulateur de mousse, agent mouillant pour surfaces dures ou en tant que milieu réactionnel pour des réactions organiques, inorganiques, bio-organiques ou photochimiques.

16. Utilisation selon la revendication 15, dans des produits lessiviels, des compositions de tensioactifs pour le nettoyage de surfaces dures, des humectants, des compositions cosmétiques, pharmaceutiques et phytosanitaires, des peintures, des produits de revêtement, des adhésifs, des dégraissants pour le cuir, des compositions pour l'industrie textile, la transformation de fibres, la transformation de métaux, l'industrie alimentaire, le traitement de l'eau, l'industrie du papier, la fermentation, la transformation de minéraux, l'ignifugation ou dans des polymérisations en émulsion.

17. Produits de lavage, de nettoyage, agents mouillants, produits de revêtement, adhésifs, dégraissants pour le cuir, humectants ou produits de traitement de textiles ou composition pharmaceutique, phytosanitaire ou cosmétique, en particulier produits pour le soin de la peau, de protection solaire ou de coiffage, gels de douche, shampooings, additifs pour le bain ou huiles essentielles, contenant, outre des composants usuels, un mélange selon l'une quelconque des revendications 1 à 6 ou une microémulsion selon l'une quelconque des revendications 9 à 14.
